# EUROPÄISCHE PATENTSCHRIFT

(11) **EP 0 612 687 B1**
(45) Veröffentlichungstag und Bekanntmachung des Hinweises auf die Patenterteilung: **20.11.1996**
(21) Anmeldenummer: 94100680.1
(22) Anmeldetag: 19.01.1994
(51) Int. Cl.: C01B 25/32, A61K 7/16

(54) **Verfahren zur Herstellung eines für den Einsatz in Zahnpasten geeigneten Dicalciumphosphat-Dihydrats**
Process for the preparation of a dicalcium phosphate dihydrate suitable for incorporation in toothpaste
Procédé de préparation d'un phosphate dicalcique dihydraté approprié pour l'incorporation dans les pâtes dentifrices

(30) Priorität: 20.02.1993 DE 4305276
(43) Veröffentlichungstag der Anmeldung: 31.08.1994
(73) Patentinhaber: HOECHST AKTIENGESELLSCHAFT, 65926 Frankfurt am Main (DE)
(72) Erfinder: Dany, Franz-Josef, Dr., D-50374 Erftstadt (DE); Kalteyer, Gerhard, D-50374 Erftstadt (DE); Nolte, Gerhard, Dr., D-50374 Erftstadt (DE); Prell, Hedwig, D-50354 Hürth (DE); Schrödter, Hermann, Dr., D-50374 Erftstadt (DE)

(56) Entgegenhaltungen:
- FR-A- 2 368 440

## Beschreibung

Die Erfindung betrifft ein Verfahren zur Herstellung von Dicalciumphosphat-Dihydrat mit guter Fluorverträglichkeit und Hydrolysestabilität, das bei seiner Anwendung in Zahnpasten eine möglichst geringe Nachdickung bewirkt, durch Umsetzung von in Wasser suspendiertem Calciumcarbonat mit einer wäßrigen Lösung von Orthophosphorsäure, Auffällung von Dimagnesiumphosphat-Trihydrat als Stabilisator durch Umsetzung wäßriger Lösungen eines Magnesiumsalzes und von Orthophosphorsäure in Gegenwart einer basischen Verbindung, sowie anschließende Zugabe von Tetranatriumpyrophosphat als weiterem Stabilisator zum Reaktionsgemisch und abschließendes Abfiltrieren, Trocknen und Mahlen des Niederschlags.

Ein derartiges "Kurzzeitverfahren" ist im wesentlichen in der DE 26 48 o61 C2 (=GB 1 548 465) beschrieben. Dicalciumphosphat-Dihydrat (DCP-D) und Dimagnesiumphosphat-Trihydrat (DMP-T) werden auch als Calciumhydrogenphosphat-Dihydrat bzw. Magnesiumhydrogenphosphat-Trihydrat bezeichnet. Die Bedeutung der Fluorverträglichkeit und Hydrolysestabilität ist in der DE 26 48 o61 C2, Spalte 2, erläutert. Das Verfahren der DE 26 48 o61 C2 befriedigt nicht bezüglich des Viscositätsverhaltens der aus dem stabilisierten DCP-D hergestellten Zahnpasten. Die Nachdickung der Zahnpasten, die mit einem derart hergestellten DCP-D zustande kommt und in hohem Maße unerwünscht ist, wird u.a. durch einen Verlust des DCP-D an Kristallwasser und durch seinen hydrolytischen Abbau zu Calciumhydroxylapatit und Orthophosphorsäure verursacht. Diese unerwünschten Reaktionen bewirken gleichzeitig in fluorhaltigen Zahnpasten über die intermediäre Bildung von Calciumfluorid und schließlich von Fluorapatit einen hohen Verlust an kariesprophylaktischen, aktiven Fluorionen.

Die unerwünschte Nachdickung läßt sich beim eingangs beschriebenen Kurzzeitverfahren weitgehend vermeiden, wenn man nach erfolgter Stabilisierung mit auf das DCP-D aufgefälltem Dimagnesiumphosphat-Trihydrat (DMP-T) erfindungsgemäß dem wäßrigen Reaktionsgemisch 0,2 bis 0,6 Gew% Tetranatriumpyrophosphat, berechnet auf das im Reaktionsgemisch niedergeschlagene Dicalciumphosphat-Dihydrat, zusetzt.

Darüberhinaus ist das Verfahren der Erfindung bevorzugt und wahlweise dadurch gekennzeichnet, daß
a) man 0,4 Gew% Tetranatriumpyrophosphat zusetzt;
b) man das Tetranatriumpyrophosphat als Pulver oder vorzugsweise in wäßriger Lösung zusetzt;
c) man annähernd stöchiometrische Mengen der Calciumcarbonnat-Suspension und der Orthophosphorsäure-Lösung in ein Reaktionsgefäß eingibt, die Reaktion bei 5o °C nicht übersteigenden Temperaturen in einem pH-Bereich von 2,2 bis 2,6 hält und das Reaktionsgemisch erst gegen Ende der Reaktion durch Zugabe restlicher Calciumcarbonat-Suspension einen pH-Wert von 3,2 bis 3,4 erreichen läßt; daß man 1o bis 2o min nachrührt, den pH-Wert des Reaktionsgemischs mit Natronlauge auf 5,6 bis 5,8 anhebt und unter gleichzeitigem Eintragen stöchiometrischer Mengen wäßriger Lösungen von Magnesiumsalz, Orthophosphorsäure und Natronlauge Dimagnesiumphosphat-Trihydrat in einer Menge von 2 bis 4 Gew%, berechnet auf das im Reaktionsgemisch niedergeschlagene Dicalciumphosphat-Dihydrat, bei einem pH-Wert von 5,6 bis 6,o auffällt, den pH-Wert mit Natronlauge auf den Neutralpunkt bringt und das Reaktionsgemisch 2 bis 3 min nachrührt; daß man dem wäßrigen Reaktionsgemisch 0,2 bis 0,6 Gew% Tetranatriumpyrophosphat, berechnet auf das im Reaktionsgemisch niedergeschlagene Dicalciumphosphat-Dihydrat, zusetzt und kurze Zeit nachrührt;
   und daß man das ausgefällte Produkt aus dem Raktionsgemisch abtrennt, trocknet und auf die für die Anwendung in Zahnpasten übliche Korngröße zerkleinert;
d) das Reaktionsgefäß zwecks Kühlung mit einem Doppelmantel versehen ist.

Als Magnesiumsalz kann z.B. Magnesiumchlorid oder -nitrat eingesetzt werden.

Die Einbringung des Tetranatriumpyrophosphats (TNPP, Na₄P₂O₇) kann in fester Form, bevorzugt jedoch in wäßriger Lösung, vorgenommen werden, weil hierdurch die offensichtlich an den Grenzflächen stattfindende, für den Stabilisierungseffekt verantwortliche topochemische Reaktion schneller und quantitativer verläuft.

Die nachfolgenden Beispiele sollen das erfindungsgemäße Verfahren zur Herstellung und Stabilisierung von DCP-D verdeutlichen.

### Beispiel 1 (Vergleichsbeispiel)

In einem rührbaren, mit Doppelmantel versehenen Edelstahlreaktor wurden 2 kg Wasser vorgelegt. Dann wurden unter intensivem Rühren mittels eines Propellers (13oo UpM) 1,o kg Calciumcarbonat als 4o gew%ige wäßrige Suspension und o,9791 kg Orthophosphorsäure als 75 gew%ige wäßrige Lösung gleichzeitig, aber bei anfangs bevorzugter Zudosierung der Orthophosphorsäure in der Weise eingetragen, daß der pH-Wert der Reaktionsmischung im Bereich von 2,2 - 2,6 gehalten wurde. Nachdem beide Reaktanten praktisch vollständig zugegeben waren, wurde die Reaktion so gesteuert, daß unter Zuführung der restlichen CaCO₃-Suspension der pH-Wert sich bei etwa 3,3 einpendelte. Die Reaktionsmischung wurde 15 min nachgerührt. Dann wurde durch Zugabe von 25 gew%iger Natronlauge der pH-Wert auf etwa 5,7 angehoben. Unter gleichzeitigem Eintragen von 0,0235 kg Magnesiumchlorid als 33,3 gew%ige Lösung, 0,0242 kg Orthophosphorsäure als 75 gew%ige wäßrige Lösung und 25 gew%iger Natronlauge wurde der pH-Wert so gesteuert, daß er einen Wert von 6 nicht überschritt. Nach vollständiger Dosierung der Reaktionspartner wurde der pH-Wert durch weitere Zugabe einer kleinen Menge Natronlauge auf den Neutralpunkt angehoben. Die Reaktionsmischung wurde weitere 2 min gerührt und dann über eine Porzellannutsche abfiltriert. Der Filterkuchen wurde mit reichlich destilliertem Wasser gewaschen, getrocknet und auf die für die Anwendung in Zahnpasten erforderliche Korngröße zerkleinert.

Die aufgefällte Menge an DMP-T betrug 0,043 kg, entsprechend 2,5 Gew%, berechnet auf 1,7194 kg ausgefälltes DCP-D.

### Beispiel 2 (Vergleichsbeispiel)

Wie Beispiel 1 mit dem Unterschied, daß 0,0376 kg Magnesiumchlorid zusammen mit 0,0387 kg H₃PO₄ in Form ihrer wäßrigen Lösungen eingebracht wurden, so daß die aufgefällte Menge an DMP-T 4 Gew% betrug.

### Beispiel 3 (erfindungsgemäß)

Wie Beispiel 1 mit dem Unterschied, daß nach Abschluß der Fällungsreaktionen dem wäßrigen Reaktionsgemisch bei einem pH-Wert von etwa 7 10,32 g Tetranatriumpyrophosphat (= 0,6 Gew% TNPP, berechnet auf 1,7194 kg ausgefälltes DCP-D) in Form einer wäßrigen Lösung unter Rühren zugegeben wurden.

### Beispiel 4 (Vergleichsbeispiel)

Wie Beispiel 1 mit dem Unterschied, daß dem gewaschenen und getrockneten Filterkuchen während der Mahlung auf die erforderliche Größe für die Anwendung in Zahnpasten 10,32 g Tetranatriumpyrophosphat (= 0,6 Gew% TNPP, berechnet auf 1,7194 kg ausgefälltes DCP-D) in Pulverform untergemischt wurden.

### Beispiel 5 (erfindungsgemäß)

Wie Beispiel 2 mit dem Unterschied, daß nach Abschluß der Fällungsreaktionen dem wäßrigen Reaktionsgemisch bei einem pH-Wert von etwa 7 3,44 g Tetranatriumpyrophosphat (= 0,2 Gew% TNPP, berechnet auf 1,7194 kg ausgefälltes DCP-D) in Form einer wäßrigen Lösung unter Rühren zugegeben wurden.

### Beispiel 6 (Vergleichsbeispiel)

Wie Beispiel 2 mit dem Unterschied, daß dem gewaschenen und getrockneten Filterkuchen während der Mahlung 6,88 g Tetranatriumpyrophosphat (= 0,4 Gew% TNPP, berechnet auf 1,7194 kg ausgefälltes DCP-D) in Pulverform untergemischt wurden.

### Beispiel 7 (erfindungsgemäß)

Wie Beispiel 2 mit dem Unterschied, daß nach Abschluß der Fällungsreaktionen dem wäßrigen Reaktionsgemisch bei einem pH-Wert von etwa 7 6,88 g Tetranatriumpyrophosphat (= 0,4 Gew% TNPP, berechnet auf 1,7194 kg ausgefälltes DCP-D) in Form einer wäßrigen Lösung unter Rühren zugegeben wurden.

### Beispiel 8 (erfindungsgemäß)

Die Durchführung der 1. Fällungsstufe erfolgte analog Beispiel 1; jedoch wurden nach Anhebung des pH-Wertes auf 5,7 gleichzeitig 0,059 kg Magnesiumnitrat als 30 gew%ige wäßrige Lösung und 0,0387 kg Orthophosphorsäure als 75 gew%igewäßrige Lösung eingetragen. Durch ebenfalls gleichzeitige Zugabe von 25 gew%iger Natronlauge wurde der pH-Wert so gesteuert, daß er einen Wert von 6 nicht überschritt. Nach Abschluß der Fällungsreaktionen wurden dem wäßrigen Reaktionsgemisch bei einem pH-Wert von etwa 7 10,32 g Tetranatriumpyro-phosphat (= 0,6 Gew% TNPP, berechnet auf 1,7194 kg ausgefälltes DCP-D) als Pulver untergerührt. Die aufgefällte Menge an DMP-T betrug 0,0688 kg, entsprechend 4 Gew%, berechnet auf 1,7194 kg ausgefälltes DCP-D.

### Beispiel 9 (Vergleichsbeispiel)

Die Herstellung erfolgte analog Beispiel 1 mit dem Unterschied, daß in der 2. Fällungsstufe 0,0705 kg Magnesiumchlorid und 0,0726 kg Orthophosphorsäure gleichzeitig zur Umsetzung kamen (entsprechend 6 Gew% DMP-T,auf DCP-D berechnet) und daß nach Abschluß der Fällungsreaktionen dem wäßrigen Reaktionsgemisch 17,19 g Tetranatriumpyrophosphat (= 1,0 Gew% TNPP, berechnet auf DCP-D) in Pulverform bei einem pH-Wert von etwa 7 untergerührt wurden.

### Beispiel 10 (Vergleichsbeispiel)

Wie Beispiel 9 mit dem Unterschied, daß die 1,0 Gew% TNPP erst dem gewaschenen und getrockneten Filterkuchen während der Mahlung in Pulverform untergemischt wurden.

### Beispiel 11 (Vergleichsbeispiel)

Wie Beispiel 2 mit dem Unterschied, daß dem wäßrigen Reaktionsgemisch bei einem pH-Wert von etwa 7 17,19 g Tetranatriumpyrophosphat (= 1,0 Gew% TNPP, berechnet auf DCP-D) in Pulverform unter Rühren zugesetzt wurden. Außerdem wurden dem gewaschenen und getrockneten Filterkuchen während der Mahlung nochmals zusätzlich 10,32 g Tetranatriumpyrophosphat (= 0,6 Gew% TNPP, berechnet auf 1,7194 kg ausgefälltes DCP-D) in Pulverform untergemischt.

Die gemäß den Beispielen 1 - 11 gefertigten Produkte wurden hinsichtlich ihres Stabilitätsverhaltens untersucht.

Die Fluorverträglichkeit der einzelnen Produkte wurde nach folgender Methode geprüft:

10 g des zu prüfenden DCP-D werden in 90 g Wasser aufgeschlämmt und auf 80°C erwärmt. Dann werden 76 mg Natriummonofluorphosphat (Na₂FPO₃, entsprechend 1000 ppm Fluor, auf eingesetztes DCP-D bezogen) eingebracht. Die Suspension wird unter ständigem Rühren genau 1 Std. bei 80°C gehalten. Es wird im Eisbad auf Zimmertemperatur abgekühlt, über eine Fritte filtriert und in einem aliquoten Teil des Filtrates der Fluorgehalt bestimmt.

Der Gehalt an löslichem Fluorion ist ein Maß für die Fluor-Verträglichkeit des DCP-D. Angegeben wird dieser in Gew% des Ausgangswertes.

Zur Bestimmung der Hydrolysestabilität kam folgende Methode zur Anwendung:

In einer Lösung von 1,63 g Natriumfluorid in 100 ml Wasser, die auf 60°C erhitzt und auf dieser Temperatur gehalten wird, werden 25 g des zu prüfenden DCP-D suspendiert und mittels eines Rührers in der Schwebe gehalten. Hierbei wird laufend der pH-Wert registriert. Es wird der Zeitpunkt ermittelt, bei dem der pH-Wert unter 4 absinkt. Die Zeit bis zum Erreichen von pH = 4 ist ein Maß für die Hydrolysestabilität des DCP-D.

Das Verhalten der Pastenviskosität wurde in der folgenden Weise untersucht:
Aus den einzelnen DCP-D-Produkten gemäß den Beispielen 1-11 wurden auf Basis folgender Formulierung Zahnpasten gefertigt:
48,0 Gew.-Teile DCP-D
24,0 Gew.-Teile Sorbit (70 gew.-%ige wäßrige Lösung)
6,0 Gew.-Teile Glycerin
1,5 Gew.-Teile Natriumlaurylsulfat
0,8 Gew.-Teile Binder
0,8 Gew.-Teile Aroma
0,76 Gew.-Teile Natriummonofluorphosphat
0,25 Gew.-Teile Tetranatriumdiphosphat
0,2 Gew.-Teile Natriumsaccharinat
Ad 100 Gew.-Teile Deionisiertes Wasser

Die auf Basis vorstehender Rezeptur hergestellten Pasten wurden in Aluminiumtuben abgefüllt und 24 Stunden bei 25°C gelagert. Bei Umgebungstemperatur wurde mittels eines Rotationsviskosimeters (Brookfield RVT DV II-Spindle D) die Viskosität in Skalenteilen (SkT) gemessen. Dann folgte eine Lagerung gerung der Pasten bei 49°C, wobei nach 3 und nach 9 Wochen die Viskositätsmessung nach Abkühlung auf Umgebungstemperatur wiederholt wurde.

Nachstehend sind zur besseren Übersicht unter Angabe der Herstellungsart die Stabilitäts- und Viskositätsergebnisse der Pasten, die aus den einzelnen DCP-D-Produkten der Beispiele 1 - 11 gefertigt wurden, tabellarisch zusammengefaßt:

| Beispiel-Nr. | 1 | 2 | 3 | 4 | 5 | 6 | 7 | 8*) | 9 | 10 | 11 |
|---|---|---|---|---|---|---|---|---|---|---|---|
| MgHPO₄·3H₂O(Gew%) | 2.5 | 4.0 | 2.5 | 2.5 | 4.0 | 4.0 | 4.0 | 4.0 | 6.0 | 6.0 | 4.0 |
| Na₄P₂O₇ (Zugabe zum Reaktionsgemisch (Gew%) | - | - | 0.6 Lsg. | - | 0.2 Lsg. | - | 0.4 Lsg. | 0.6 Plv. | 1.0 Plv. | - | 1.0 Plv. |
| Na₄P₂O₇ (Zugabe in Mühle) Gew% | - | - | - | 0.6 | - | 0.4 | - | - | - | 1.0 | 0.6 |
| Fluor-Verträglichkeit; Gew% wasserlösl.Fluor | 71 | 75 | 80 | 80 | 78 | 80 | 80 | 80 | 82 | 82 | 81 |
| Hyrolysestabilität Zeit (h) bis Erreichen von pH = 4 | 8.3 | 9.4 | 11.0 | 11.5 | 10.0 | 10.5 | 10.0 | 10.5 | 17.0 | 18.0 | 12.0 |

| **Pastenviskosität (Skt)** | | | | | | | | | | | |
|---|---|---|---|---|---|---|---|---|---|---|---|
| nach 24 h bei bei 25°C | 23 | 25 | 29 | 39 | 30 | 29 | 30 | 30 | 29 | 27 | 35 |
| nach 3 Wochen bei 49°C | 72 | 70 | 41 | 80 | 64 | 84 | 36 | 38 | 70 | 82 | >100 |
| nach 9 Wochen bei 49°C | >100 | >100 | 55 | >100 | 60 | >100 | 52 | 56 | >100 | >100 | >100 |
| Lsg. = Lösung; Plv. = Pulver; Skt = Skalenteile | | | | | | | | | | | |

| | | | | | | | | | | | |
|---|---|---|---|---|---|---|---|---|---|---|---|
| *) Als Mg-Träger wurde Mg(NO₃)₂ eingesetzt im Gegensatz zu den anderen Beispielen, wo Mg(Cl)₂ verwendet wurde. | | | | | | | | | | | |

Wie ersichtlich liefern die erfindungsgemäßen Produkte, die gemäß den Beispielen 3, 5, 7 und 8 hergestellt sind, die mit Abstand besten Ergebnisse in Bezug auf das Viskositätsverhalten der daraus hergestellten Zahnpasten, wobei gleichzeitig auch gute Werte für die Fluorverträglichkeit und die Hydrolysestabilität festgestellt werden.

## Patentansprüche

1. Verfahren zur Herstellung von Dicalciumphosphat-Dihydrat mit guter Fluorverträglichkeit und Hydrolysestabilität, das bei seiner Anwendung in Zahnpasten eine möglichst geringe Nachdickung bewirkt, durch Umsetzung von in Wasser suspendiertem Calciumcarbonat mit einer wäßrigen Lösung von Orthophosphorsäure, Auffällung von Dimagnesiumphosphat-Trihydrat als Stabilisator durch Umsetzung wäßriger Lösungen eines Magnesiumsalzes und von Orthophosphorsäure in Gegenwart einer basischen Verbindung sowie anschließende Zugabe von Tetranatriumpyrophosphat als weiterem Stabilisator zum Reaktionsgemisch und abschließendes Abfiltrieren, Trocknen und Mahlen des Niederschlags, dadurch gekennzeichnet, daß man dem wäßrigen Reaktionsgemisch 0,2 bis 0,6 Gew% Tetranatriumpyrophosphat, berechnet auf das im Reaktionsgemisch niedergeschlagene Dicalciumphosphat-Dihydrat, zusetzt.

2. Verfahren nach Anspruch 1, dadurch gekennzeichnet, daß man das Tetranatriumpyrophosphat als Pulver oder vorzugsweise in wäßriger Lösung zusetzt.

3. Verfahren nach Anspruch 1 oder 2, dadurch gekennzeichnet, daß man annähernd stöchiometrische Mengen der Calciumcarbonat-Suspension und der Orthophosphorsäure-Lösung in ein Reaktionsgefäß eingibt, die Reaktion bei 5o °C nicht übersteigenden Temperaturen in einem pH-Bereich von 2,2 bis 2,6 hält und das Reaktionsgemisch erst gegen Ende der Reaktion durch Zugabe restlicher Calciumcarbonat-Suspension einen pH-Wert von 3,2 bis 3,4 erreichen läßt; daß man 1o bis 2o min nachrührt, den pH-Wert des Reaktionsgemischs mit Natronlauge auf 5,6 bis 5,8 anhebt und unter gleichzeitigem Eintragen stöchiometrischer Mengen wäßriger Lösungen von Magnesiumsalz, Orthophosphorsäure und Natronlauge Dimagnesiumphosphat-Trihydrat in einer Menge von 2 bis 4 Gew%, berechnet auf das im Reaktionsgemisch niedergeschlagene Dicalciumphosphat-Dihydrat, bei einem pH-Wert von 5,6 bis 6,o auffällt, den pH-Wert mit Natronlauge auf den Neutralpunkt bringt und das Reaktionsgemisch 2 bis 3 min nachrührt; daß man dem wäßrigen Reaktionsgemisch 0,2 bis 0,6 Gew% Tetranatriumpyrophosphat, berechnet auf das im Reaktionsgemisch niedergeschlagene Dicalciumphosphat-Dihydrat, zusetzt und kurze Zeit nachrührt; und daß man das ausgefällte Produkt aus dem Reaktionsgemisch abtrennt, trocknet und auf die für die Anwendung in Zahnpasten übliche Korngröße zerkleinert.

4. Verfahren nach einem der vorhergehenden Ansprüche, dadurch gekennzeichnet, daß man 0,4 Gew% Tetranatriumpyrophosphat zusetzt.

5. Verfahren nach Anspruch 3 oder 4, dadurch gekennzeichnet, daß das Reaktionsgefäß zwecks Kühlung mit einem Doppelmantel versehen ist.

## Claims

1. A process for the preparation of dicalcium phosphate dihydrate, which has a good compatibility with fluorine and stability to hydrolysis and causes the minimum possible after-thickening when used in toothpastes, by reaction of calcium carbonate suspended in water with an aqueous solution of orthophosphoric acid, precipitation of dimagnesium phosphate trihydrate, as a stabilizer, by reaction of aqueous solutions of a magnesium salt and of orthophosphoric acid in the presence of a basic compound and subsequent addition of tetrasodium pyrophosphate to the reaction mixture as a further stabilizer, and final filtering, drying and grinding of the precipitate, which comprises adding to the aqueous reaction mixture 0.2 to 0.6 % by weight of tetrasodium pyrophosphate calculated with respect to the dicalcium phosphate dihydrate precipitated in the reaction mixture.

2. The process as claimed in claim 1, wherein the tetrasodium pyrophosphate is added as powder or preferably in aqueous solution.

3. The process as claimed in claim 1 or 2, wherein approximately stoichiometric amounts of the calcium carbonate suspension and the orthophosphoric acid solution are introduced into a reaction vessel, the reaction is kept at temperatures which do not exceed 50°C in a pH range from 2.2 to 2.6 and the reaction mixture is allowed to reach a pH of 3.2 to 3.4 by addition of the remaining calcium carbonate suspension only towards the end of the reaction; wherein the mixture is subsequently stirred for 10 to 20 minutes, the pH of the reaction mixture is increased to 5.6 to 5.8 with sodium hydroxide solution and dimagnesium phosphate trihydrate is precipitated in an amount of 2 to 4 % by weight, calculated with respect to the dicalcium phosphate dihydrate precipitated in the reaction mixture, at a pH of 5.6 to 6.0 while simultaneously introducing stoichiometric amounts of aqueous solutions of magnesium salt, orthophosphoric acid and sodium hydroxide solution, the pH is brought to the neutral point with sodium hydroxide solution and the reaction mixture is subsequently stirred for 2 to 3 minutes; wherein 0.2 to 0.6 % by weight of tetrasodium pyrophosphate, calculated with respect to the dicalcium phosphate dihydrate precipitated in the reaction mixture, is added to the aqueous reaction mixture and the mixture is subsequently stirred for a short time; and wherein the precipitated product is separated off from the reaction mixture, dried and comminuted to the particle size customary for use in toothpastes.

4. The process as claimed in any one of the preceding claims, wherein 0.4 % by weight of tetrasodium pyrophosphate is added.

5. The process as claimed in claim 3 or 4, wherein the reaction vessel is provided with a double jacket for the purpose of cooling.

## Revendications

1. Procédé de préparation d'un phosphate dicalcique dihydraté bien compatible avec le fluor et bien stable à l'hydrolyse donnant, à l'utilisation dans les pâtes dentifrices, un épaississement aussi faible que possible, par réaction du carbonate de calcium en suspension dans l'eau avec une solution aqueuse d'acide orthophosphorique, précipitation de phosphate dimagnésien trihydraté, servant de stabilisant, par réaction de solutions aqueuses d'un sel de magnésium et d'acide orthophosphorique en présence d'un composé basique et addition subséquente au mélange de réaction de pyrophosphate tétrasodique, autre stabilisant, en terminant par une filtration, un séchage et un broyage du précipité, caractérisé en ce que l'on ajoute au mélange de réaction aqueux de 0,2 à 0,6 % en poids de pyrophosphate tétrasodique par rapport au phosphate dicalcique dihydraté précipité dans le mélange de réaction.

2. Procédé selon la revendication 1, caractérisé en ce que le pyrophosphate tétrasodique est ajouté à l'état de poudre ou, de préférence, de solution aqueuse.

3. Procédé selon la revendication 1 ou 2, caractérisé en ce que l'on introduit dans un récipient de réaction des quantités à peu près stoechiométriques de la suspension de carbonate de calcium et de la solution d'acide orthophosphorique, on maintient le mélange de réaction à des températures ne dépassant pas 50°C dans l'intervalle de pH de 2,2 à 2,6 et on porte le pH du mélange de réaction à un pH de 3,2 à 3,4 que vers la fin de la réaction, par addition du restant de la suspension de carbonate de calcium; en ce que l'on agite pendant encore 10 à 20 minutes, on relève le pH du mélange de réaction à un niveau de 5,6 à 5,8 à l'aide de lessive de soude et, par addition simultanée de quantités stoechiométriques d'une solution aqueuse de sel de magnésium, d'acide orthophosphorique et de lessive de soude, on précipite à un pH de 5,6 à 6,0 du phosphate dimagnésien trihydraté en quantité de 2 à 4 % en poids, par rapport au phosphate dicalcique dihydraté précipité dans le mélange de réaction, on porte le pH à neutralité par la lessive de soude et on agite le mélange de réaction pendant encore 2 à 3 minutes ; en ce que l'on ajoute au mélange de réaction de 0,2 à 0,6 % en poids de pyrophosphate tétrasodique par rapport au phosphate dicalcique dihydraté précipité dans le mélange de réaction et on agite ensuite pendant un court moment ; et en ce que l'on sépare le produit précipité du mélange de réaction, on le sèche et on le broie à la dimension de grain habituelle pour l'utilisation dans des pâtes dentifrices.

4. Procédé selon une des revendications qui précèdent, caractérisé en ce que l'on ajoute 0,4 % en poids de pyrophosphate tétrasodique.

5. Procédé selon la revendication 3 ou 4, caractérisé en ce que le récipient de réaction est équipé d'une double enveloppe permettant le refroidissement.
